(19) Europäisches Patentamt
European Patent Office
Office européen des brevets

(11) Veröffentlichungsnummer: **0 252 216 B1**

(12) **EUROPÄISCHE PATENTSCHRIFT**

(45) Veröffentlichungstag der Patentschrift: **29.01.92**

(51) Int. Cl.⁵: **C12N 9/80**

(21) Anmeldenummer: **87103662.0**

(22) Anmeldetag: **13.03.87**

(54) **Mikrobiologisch hergestellte alpha-Acetylaminozimtsäure-Acylase, Verfahren zu ihrer Gewinnung und ihre Verwendung.**

(30) Priorität: **28.06.86 DE 3621839**

(43) Veröffentlichungstag der Anmeldung:
**13.01.88 Patentblatt 88/02**

(45) Bekanntmachung des Hinweises auf die Patenterteilung:
**29.01.92 Patentblatt 92/05**

(84) Benannte Vertragsstaaten:
**BE CH DE FR GB LI NL SE**

(56) Entgegenhaltungen:

**CHEMICAL ABSTRACTS, Band 101, Nr. 13, 24. September 1984, Seite 502, Zusammenfassung Nr. 108947g, Columbus, Ohio, US; & JP-A-59 74 994 (TANABE SEIYAKU CO., LTD) 27-04-1984**

**CHEMICAL ABSTRACTS, Band 97, Nr. 21, 22. November 1982, Seite 627, Zusammenfassung Nr. 180148p, Columbus, Ohio, US; & JP-A-82 99 198 (TANABE SEIYAKU CO., LTD) 10-06-1982**

(73) Patentinhaber: **Degussa AG**
**Wolfgang Rodenbacher Chaussee 4**
**W-6450 Hanau 1(DE)**

Patentinhaber: **Gesellschaft für Biotechnologische Forschung mbH (GBF)**
**Mascheroder Weg 1**
**W-3300 Braunschweig-Stöckheim(DE)**

(72) Erfinder: **Kula, Maria-Regina, Prof. Dr.**
**Selgenbusch 12**
**W-5162 Niederziehr-Hambach(DE)**
Erfinder: **Hummel, Werner, Dr.**
**Claudiusstrasse 11**
**W-5177 Titz(DE)**
Erfinder: **Schütte, Horst, Chem.-Ing.**
**Nordring 29 a**
**W-3320 Salzgitter 51(DE)**
Erfinder: **Leuchtenberger, Wolfgang, Dr.**
**Geschw.-Scholl-Strasse 1**
**W-6454 Bruchköbel(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**CHEMICAL ABSTRACTS, Band 68, Nr. 5, 29. Januar 1968, Seite 1882, Zusammenfassung Nr. 19748a, Columbus, Ohio, US; Y. KAMEDA et al.: "Acylase activity and microorganisms. XXII. Isolation of soil bacteria capable of producing delta-ornithine acylase (5-N-acylornithine amidohydrolase)", & CHEM. PHARM. BULL. (TOKYO) 15(10), 1573-7(1967)**

## Beschreibung

Die Erfindung betrifft ein bisher nicht beschriebenes Enzym, das Umsetzungen folgender Art katalysiert:

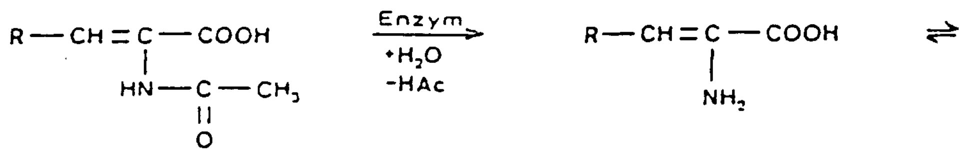


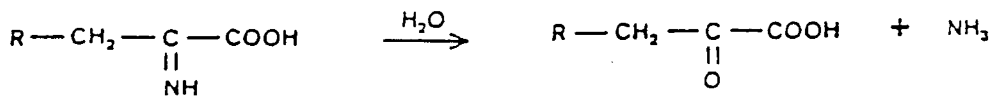


Einen besonders guten Umsatz erhält man mit R = $C_6H_5$-. Diese Verbindung, α-Acetylaminozimtsäure-Acylase ist gekennzeichnet durch die folgenden Eigenschaften:

1) Reaktivität:
sie reagiert mit α-Acetylaminozimtsäure unter Bildung von α-Imino-β-phenyl-propionsäure bzw. Phenylbrenztraubensäure;

2) Subtratspezifität:
sie hydrolysiert α-Acetylaminozimtsäure, nicht aber andere α-Acetylaminocarbonsäuren, wie α-Acetylaminoacrylsäure oder N-Acetylphenylalanin;

3) Optimaler pH-Wert:
der optimale pH-Wert ist 7,5 ± 1;

4) pH-Stabilität:
sie zeigt eine gute Stabilität im pH-Bereich zwischen 6,9 und 9,4 (mehr als 90 % Restaktivität nach 2 Wochen bei 4 °C);

5) Optimale Temperatur:
die optimale Temperatur beträgt 52 °C bei einem pH-Wert von 7,5;

6) Temperaturbeständigkeit:
bei 55 °C sind nach 30-minütiger Inkubation noch 80 % Restaktivität nachweisbar;

7) Einflüsse von Inhibitoren und Aktivatoren:
inhibierend wirken vor allem Quecksilber-Verbindungen (p-Mercuribenzoat und $Hg_2Cl_2$), $CdCl_2$ und o-Phenanthrolin, Zusatz von Dithiothreitol (1 mMol) aktiviert das Enzym;

8) Molekulargewicht:
das Molekulargewicht beträgt 50 000 ± 5000 Dalton;

9) Untereinheiten:
das Molekül besteht aus 2 gleichgroßen Untereinheiten mit je 26 000 ± 3000 Dalton;

10)der $K_M$-Wert für das Substrat α-Acetylaminozimtsäure bei pH 7,5 ist 0,45 mMol.

Die erfindungsgemäße α-Acetylaminozimtsäure-Acylase kann mittels zweier Brevibakterium-Stämme gewonnen werden, die am 14.02.1986 bei der National Collection of Industrial Bacteria in Aberdeen (Schottland) unter den Nummern NCIB 12246 und NCIB 12247 hinterlegt wurden.

Die folgenden Eigenschaften zeigen, daß die beiden Mikroorganismen zur Gattung Brevibacterium gehören:

Sie wachsen in kurzen grampositiven Stäbchen, die mit zunehmendem Alter in kokkoide Formen übergehen. Die Zellen sind unbeweglich und bilden keine Sporen. Wachstum erfolgt strikt aerob. Aus Glucose wird keine Säure gebildet. Katalasereaktion und Nitratreduktion sind positiv, Harnstoffspaltung positiv, Gelatine-, Casein- und Stärkeabbau negativ, $H_2S$-Bildung negativ, Wachstum bei 41 °C negativ. Als Zellwandzucker wurden Arabinose, Mannose und Galactose nachgewiesen. Die Mikroorganismen können aufbewahrt werden als lyophilisierte Kultur, durch Einfrieren bei -80 °C oder in flüssigem Stickstoff bei -196 °C. Arbeitskulturen werden auf Schrägagar-Röhrchen (ACA-Medium) gehalten.

Zur Gewinnung der erfindungsgemäßen α-Acetylaminozimtsäure-Acylasewird Brevibacterium spec.

NCIB 12246 oder NCIB 12247 in einem wäßrigen Nährmedium, das eine Quelle für Kohlenstoff und Stickstoff, Thiamin, Mineralsalze und α-Acetylaminozimtsäure als Induktor enthält, bei einem Ausgangs-pH-Wert zwischen 6,0 und 8,0 und einer Temperatur zwischen 25 °C und 35 °C aerob kultiviert, die Zellmasse abgetrennt und das Enzym aus den Zellen isoliert.

Größere Mengen des Enzyms können beispielsweise so gewonnen werden, daß man Brevibacterium spec. NCIB 12246 oder NCIB 12247 in an sich bekannter Weise in einem Bioreaktor im gewünschten Maßstab, z.B. im 10 l-Maßstab, anzieht. Für eine erfolgreiche Anzucht sind wichtig:

- eine gute Belüftung (obligat aerober Organismus);
- ein Ausgangs-pH-Wert im Medium zwischen 6,0 und 8,0;
- die Anwesenheit von Mineralsalzen (z.B. in komplexer Form als Maisquellwasser);
- die Anwesenheit von geringen Mengen an Thiamin (1 bis 2 µg/l) und
- die Anwesenheit von ACA (0,2 bis 0,4 Gewichtsprozent) im Medium zur Induktion des Enzyms.

Die Isolierung des Enzyms ist nach Aufschluß der Zellen durch die Kombination an sich bekannter Methoden der Enzymreinigung möglich. Es kann als Bestandteil eines gekoppelten Enzymsystems für enzymatische Umsetzungen verwendet werden, die über die Zwischenstufe α-Imino-β-phenylpropionsäure bzw. Phenylbrenztraubensäure verlaufen. So kann man es beispielsweise mit einer geeigneten Dehydrogenase kombinieren, um ACA in eine entsprechende α-Aminocarbonsäure oder α-Hydroxycarbonsäure umzuwandeln. Kombiniert mit einer L-Phenylalanin-Dehydrogenase wandelt es z.B. ACA in L-Phenylalanin um, kombiniert mit einer L-2-Hydroxy-4-methylpentansäure-Dehydrogenase in L-Phenylmilchsäure und kombiniert mit einer D-2-Hydroxy-4-methylpentansäure-Dehydrogenase in D-Phenylmilchsäure.

Die Erfindung soll durch die nachfolgenden Beispiele näher erläutert werden. Prozentangaben bedeuten, sofern nicht anders angegeben, Gewichtsprozente.

Beispiel 1: Screening auf α-Acetylaminozimtsäure-Acylase-Produzenten

10 Bodenproben aus der Umgebung von Braunschweig wurden mit steriler Salzlösung (0,9 % NaCl) aufgeschlemmt, in üblicher Weise verdünnt und Aliquots des wäßrigen überstandes auf Petrischalen mit festem Nährboden ausgespatelt. Der Nährboden hatte folgende Zusammensetzung (ACA-Medium):

| | |
|---|---|
| α-Acetylaminozimtsäure (ACA) | 5 g |
| $(NH_4)_2SO_4$ | 0,25 g |
| $K_2HPO_4$ | 2 g |
| Hefeextrakt | 0,2 g |
| Spurenelemente-Lösung | 1 ml |
| $MgSO_4 \bullet 7 H_2O$ | 100 mg |
| $CaCO_3$ | 20 mg |
| Destilliertes Wasser | 1 l |
| Agar | 20 g |
| pH-Wert | 7,5 |

Das Medium wurde durch Autoklavieren sterilisiert, nach dem Abkühlen wurden 1 ml sterilfiltrierte Vitaminlösung pro 1 l Medium sowie 50 mg Cycloheximid (Actidione) pro 1 l zugesetzt (Zusammensetzung der Vitaminlösung nach Schlegel, H. G. "Allgemeine Mikrobiologie", Thieme Verlag, S. 169, 1981) und der Nährboden in sterile Petrischalen gegossen.

Die beimpften Platten wurden 3 bis 4 Tage bei 27 °C bebrütet. Nach Wachstum der Mikroorganismen wurde das Koloniemuster von Agarplatten mit einer geeigneten Koloniezahl (50-200) steril auf einen Samtstempel abgeimpft, dann auf 2 Platten überstempelt, die unterschiedliche Agarnährböden enthielten: Eine Platte enthielt das oben angeführte ACA-Medium, die andere ein Phenylalaninhaltiges Nährmedium mit folgender Zusammensetzung (Phenylalanin-Medium):

| | |
|---|---|
| L-Phenylalanin | 10 g |
| $KH_2PO_4$ | 2 g |
| Hefeextrakt | 0,2 g |
| Agar | 20 g |
| Destilliertes Wasser | 1 L |

Der pH-Wert wurde auf 7,2 eingestellt.

Die Platten wurden erneut 3 bis 5 Tage bei 27 °C bebrütet. Durch Vergleich der Koloniemuster konnten diejenigen Organismen herausgesucht werden, die sowohl auf dem ACA- als auch auf dem Phenylalanin-Medium wachsen können. Diese wurden abgeimpft und im folgenden auf ACA-Medium gehalten.

Die isolierten Mikroorganismen wurden dann in üblicher Weise auf Reinheit überprüft (Verdünnungsausstrich, Mikroskopieren). Einheitlich erscheinende Stämme wurden sodann in 100 ml

Flüssigmedium (500 ml Erlenmeyer-Kolben mit 2 Schikanen) bei 27 °C auf der Rundschüttelmaschine bei 120 UpM vermehrt. Das Flüssigmedium hatte folgende Zusammensetzung:

| | |
|---|---|
| α-Acetylaminozimtsäure (ACA) | 3 g |
| $(NH_4)_2SO_4$ | 0,25 g |
| Hefeextrakt | 1 g |
| Maisquellwasser (Trockenpulver) | 5 g |
| $K_2HPO_4$ | 2 g |
| Destilliertes Wasser | 1 l |
| pH | 7,5 |

Nach 2 bis 3 Tagen wurde der Inhalt des Schüttelkolbens zentrifugiert (20 Minuten bei 10 000 g in einer Kühlzentrifuge) und die sedimentierten Zellen wurden in 0,05 M Kaliumphosphatpuffer (pH 7,4) suspendiert (4 ml Puffer pro 1 g Bakterien-Feuchtmasse).

Die Mikroorganismen in dieser Suspension müssen dann in üblicher Weise aufgeschlossen (z.B. Schütteln mit feinen Glasperlen, Ultraschall-Behandlung, Frenchpresse) werden. Wir haben die Suspension mit Glasperlen (0,1 bis 0,2 mm ϕ) versetzt, wobei pro 1 ml Zellsuspension 2 g Glasperlen verwendet wurden, und dann in einem Reagenzglas für 10 Minuten mit einem Laborschüttler (Typ Reax 1-D-R, Firma Heidolph) gemixt. Unter diesen Bedingungen konnten die meisten Isolate gut aufgeschlossen werden. Unlösliche Zellbestandteile und die Glasperlen wurden durch Zentrifugation abgetrennt (2 Minuten mit 12 000 UPM in einer Eppendorf-Tischzentrifuge). Der Überstand wurde dann als Enzymquelle (Rohextrakt) getestet. Zum Nachweis des Enzyms wurde ein photometrischer Test benutzt.

Der Testansatz enthielt:
0,7 M Ammoniumchlorid/Ammoniakpuffer für pH 9,2
0,1 mM NADH
10 mM ACA
1 U/ml L-Phenylalanin-Dehydrogenase und limitierende Mengen an Acylase (1-20 µg Protein pro Test).

Die Abnahme der Extinktion von NADH bei 340 nm wurde gemessen. Von den erhaltenen Werten wurde ein Nullwert abgezogen, den man erhielt, wenn der Test ohne Zugabe von ACA ablief. Die Enzymaktivität wird in internationalen Einheiten angegeben, wobei eine Einheit die Abnahme von 1µMol NADH/min bedeutet.

Die höchste Aktivität bei diesem Test zeigten die Stämme Brevibacterium spec. 37/3 (NCIB 12246) und 38/3 (NCIB 12247). Für die Produktion der Acylase in größerem Maßstab wurde der Stamm 37/3 eingesetzt.

Beispiel 2: Produktion der α-Acetylaminozimtsäure-Acylase

a) Anzucht von Brevibacterium spec. 37/3 im 10 l-Maßstab

Es wurde ein Bioreaktor verwendet, der mit 10 l Medium gefüllt war. Das Medium enthielt pro 1 l:
3 g ACA
0,25 g $(NH_4)_2SO_4$
1 g Hefeextrakt
10 g Maisquellwasser (Trockenpulver)
2 g $K_2HPO_4$

Der pH-Wert betrug 7,5.

Nach Sterilisation wurde das Medium mit 300 ml einer Vorkultur angeimpft, die 48 Stunden im gleichen Medium angezogen worden war. Die Wachstumsbedingungen im Fermenter waren:

| | |
|---|---|
| Temperatur: | 30 °C |
| Belüftungsrate | 60 l Luft/Stunde |

Turbinenrührer mit 250 UpM.

Zu verschiedenen Zeiten des Wachstums wurden Proben (100 ml) entnommen und nach einem Test auf enzymatische Aktivität in diesen Zellen der maximal erreichbare Enzymgehalt bzw. der günstigste Erntezeitpunkt bestimmt. Als Maß für das Wachstum der Kultur wurde zudem die Trübung (optische Dichte) bei 578 nm gemessen. Es zeigte sich, daß die Hauptmenge an Acylase dann gebildet ist, wenn der Organismus weitgehend ausgewachsen ist. Nach Erreichen eines maximalen Wertes sinkt die Enzymaktivität relativ schnell wieder ab.

b) Gewinnung des Rohextraktes

Die Bakterien-Feuchtmasse (230 g) wurde in 20 mM Phosphatpuffer (pH 7,5)unter Zusatz von 0,1 % 2-Mercaptoäthanol suspendiert, so daß die Konzentration der Zellsuspension 40 %ig war (Endvolumen hier 575 ml). Die Zell-Inhaltstoffe wurden aus der gekühlten Suspension (~4 °C) durch einen mechanischen Zellaufschluß in einer Glasperlenmühle der Firma Bachofen (Dyno-Mill, Typ KDL) freigesetzt. Hierbei wurde der 300 ml fassende Mahlbehälter mit 0,25-0,5 mm großen Glasperlen gefüllt, so daß sich ein Schüttvolumen von 255 ml ergab (85 %). Der Aufschluß wurde bei einer Rührwellendrehzahl von 3000 UpM und einer Durchflußrate von 5 l/h durchgeführt. Der Kühlmantel des Mahlbehälters sowie das Rührwellenlager wurden während des Laufens mit Äthylenglycollösung von -20 °C gekühlt, um eine Erwärmung des Produktes weitgehend zu vermeiden. Nach 3 Durchläufen war ein Desintegrationsgrad von 90 % erreicht. Der pH-Wert der Suspension wurde nach der Homogenisation mit Kalilauge auf pH 7,5 eingestellt.

c) Flüssig-Flüssig-Verteilung

Mit dem ersten Verteilungsschritt sollen die Zellbruchstücke aus dem Rohextrakt gemäß DE-PS 26 39 129 abgetrennt werden. Zu diesem Zweck wurde ein wäßriges Zwei-Phasensystem hergestellt, welches 10 % (w/w) Polyäthylenglykol 6000, 7 % (w/w) Phosphatpuffer für pH 8,0, 0,05 M Natriumchlorid und 575 ml Rohextrakt in einem 1,15 kg-System enthielt. Um das Gleichgewicht der Verteilung zu erreichen, wurde d-s Zwei-Phasensystem eine Stunde lang gerührt und anschließend durch Zentrifugation getrennt.

Die Oberphase (745 ml) enthielt über 90 % der ACA-Acylase. Die Unterphase enthielt Zellbruchstücke sowie unter diesen Bedingungen in die Unterphase extrahierte Proteine und wurde verworfen. Die enzymhaltige Oberphase wurde mit 8 % (w/v) Phosphatpuffer für pH 6,0 und 0,6 M Natriumchlorid, berechnet auf ein Endvolumen von 1,5 l, versetzt und 1 Stunde gerührt. Das sich ausbildende Polyäthylenglykol/Salz-System ließ sich in einem Absetzgefäß in etwa 1 Stunde vollständig trennen. Bei diesem Verteilungsschritt wurde die ACA-Acylase nahezu vollständig in die Unterphase extrahiert. Die Trennung der Phasen erfolgte durch Ablassen der Unterhphase (1040 ml).

d) Chromatographie an Phenyl-Sepharose Cl-4B

Die salzreiche Unterphase, die die ACA-Acylase enthielt, wurde direkt an der hydrophoben Phenyl-Sepharose Cl-4B chromatographiert. Der Vorteil dieses Verfahrens liegt darin, daß eine zeitraubende Dialyse der salzreichen Unterphase vor dem Chromatographieschritt entfällt Die Phenyl-Sepharose Cl-4B war gegen einen Puffer äuquilibriert worden, der 50 mM Phosphatpuffer für pH 7,5, 0,1 % (v/v) 2-Mercaptoäthanol und 1 M Ammoniumsulfat enthielt (Startpuffer).Die Unterphase des zweiten Verteilungsschritts wurde auf eine 5 × 11 cm Säule aufgetragen und mit zwei Säulenvolumen an Startpuffer nachgewaschen. Die ACA-Acylase wurde danach mit einem 1 mM Phosphatpuffer für pH 7,5 eluiert.

e) Fast Protein Liquid Chromatography (FPLC) an Mono Q

Das Eluat wurde über einen Super Loop auf eine 0,5 × 5 cm Mono Q-Säule (FPLC - System der Firma Pharmacia, Uppsala, Schweden) aufgetragen und bei einem Druck von maximal 40 bar chromatographiert. Für die Proteinmenge von 58 mg waren 2 Läufe notwendig. Der Anionenaustauscher war zuvor gegen einen 10 mM Phosphatpuffer für pH 7,5 äquilibriert worden (Startpuffer).

Die ACA-Acylase wurde mit einem linearen 20 ml Gradienten, der 0,1 bis 0,4 M Natriumchlorid in Startpuffer enthielt, eluiert. Die Elution erfolgte von 0,25 bis 0,3 M Natriumchlorid. Die Zeit einer solchen Chromatograhie ist mit etwa 20 Minuten anzusetzen.

f) Fast Protein Liquid Chromatography (FPLC) an Superose 12

Die mit Superose 12 gefüllte Säule (HR 10/30) war gegen einen Puffer äquilibriert,der 50 mM Phosphatpuffer für pH 7,5 und 0,15 M Natriumchlorid enthielt. Das Eluat von der Q-Säule war zunächst in einem Minicon-Konzentrator der Fa. Amicon auf ein Volumen von 500 µl konzentriert und dann in 2 Läufen mit jeweils 250 µl Probemenge an Superose 12 gelfiltriert worden. Die Zeit für eine Gelfiltration an Superose 12 (HR 10/30-Säule; Druck ~30 bar) beträgt etwa 40 Minuten.

Die aktiven Fraktionen wurden vereinigt, mit 50 % (w/v) Glycerin versetzt und bei -20 °C eingefroren.

Die Ergebnisse der Aufreinigung sind in Tabelle 1 beschrieben.

Tabelle 1: Reinigung der ACA-Acylase

| Reinigungsschritt | Volumen | Protein | Totalaktivität | spez. Aktivität | Ausbeute | Anreicherung |
|---|---|---|---|---|---|---|
| | ml | mg | U | U/mg | % | -fach |
| Rohextrakt | 575 | 1 944 | 500 | 0,26 | 100 | 1 |
| Oberphase | 745 | 521 | 455 | 0,87 | 91 | 3,3 |
| Unterphase | 1 040 | 416 | 415 | 1,0 | 83 | 3,8 |
| Phenyl-Sepharose Cl-4B | 197 | 58 | 345 | 5,9 | 69 | 23.1 |
| FPLC-Mono Q | 4,3 | 17 | 303 | 17,8 | 61 | 68,5 |
| FPLC-Superose S 12 | 3,6 | 8,4 | 240 | 28,6 | 48 | 110 |

Test mit D-2- Hydroxy-4-methylpentansäure-Dehydrogenase

Reinigung aus 230 g Bakterien-Feuchtmasse

Beispiel 3: Induktion der α-Acetylaminozimtsäure-Acylase

a) Veränderung des Anzuchtmediums

Die Acylase wird gebildet, wenn der Stamm Brevibacterium spec. 37/3 in einem Medium mit ACA als C- und N-Quelle angezogen wird. Die Enzymausbeuten (U/g ACA) sind allerdings relativ niedrig. Wie die Tabelle 2 zeigt, kann die Ausbeute gesteigert werden, wenn das Medium zusätzlich geeignete Stickstoffhaltige Verbindungen enthält. C-Quellen wie Glycerin oder Glukose unterdrücken die Acylase-Bildung.

Tabelle 2

| Acylase-Aktivität in Abhängigkeit von der Zusammensetzung des Mediums (Alle Medien enthielten jeweils 2 g/l $KH_2PO_4$, Vitamine + Spurenelemente; der pH war 7,5) | | |
|---|---|---|
| Medium g/l | U/l | mU/mg |
| ACA (5) | 33 | 60 |
| ACA (5) + $(NH_4)_2SO_4$ (0,25) | 80 | 90 |
| ACA (5) + Hefeextrakt (1) | 40 | 70 |
| ACA (5) + Maisquellwasser (5) | 100 | 95 |
| ACA (5) + Maisquellwasser (5) + $(NH_4)_2SO_4$ (0,25) | 110 | 95 |
| ACA (5) + Glycerin (10) | 0 | 0 |
| ACA (5) + Glukose (10) | 0 | 0 |

b) Veränderung der ACA-Konzentration

Zu einem Grundmedium von
5 g Maisquellwasser
0,25 g $(NH_4)_2SO_4$
2 g $KH_2PO_4$
1 ml Vitaminlösung
1 ml Spurenelemente-Lösung
1 l $H_2O$
wurden unterschiedliche Konzentrationen an ACA zugesetzt. Tabelle 3 zeigt, daß man eine gute Aktivität im Rohextrakt erhält, wenn das Medium 2 bis 5 g ACA pro l enthält.

Tabelle 3

| Enzymgehalt bei Anzucht in Gegenwart von steigenden Mengen ACA | | |
|---|---|---|
| α-Acetylaminozimtsäure (g/l) | Acylase | |
| | U/l | U/mg |
| ohne | 0 | 0 |
| 0,5 | 39 | 0,036 |
| 1 | 51 | 0,068 |
| 2 | 95 | 0,100 |
| 3 | 112 | 0,108 |
| 4 | 136 | 0,116 |
| 5 | 169 | 0,105 |

Beispiel 4: Abhängigkeit der Reaktionsgeschwindigkeit vom pH-Wert

Die Reaktionsgeschwindigkeit der hydrolytischen Abspaltung von Essigsäure aus der Verbindung α-Acetylaminozimtsäure in Gegenwart der ACA-Acylase und der nachfolgenden reduktiven Aminierung des entstandenen Phenylpyruvates in Gegenwart von L-Phenylalanin-Dehydrogenase zu L-Phenylalanin oder der Hydrogenierung des Phenylpyruvates in Gegenwart von D-2-Hydroxy-4-methylpentansäure-Dehydrogenase zu D-Phenylmilchsäure wurde in Abhängigkeit vom pH-Wert der Reaktionslösung untersucht.

Der Testansatz hatte in Verbindung mit der L-Phenylalanin-Dehydrogenase folgende Zusammensetzung
0,23 mM NADH

16 mM ACA
0,5 U L-Phenylalanin-Dehydrogenase
0,7 M Ammoniumchloridlösung bei unterschiedlichen pH-Werten und limitierende Mengen an ACA-Acylase.

Die gewählten pH-Werte zwischen pH 7,5 und 10,3 wurden durch Zugabe von Ammoniak bzw. Salzsäure zu der 0,7 M Ammoniumchloridlösung vor Zusammenmischen des Testansatzes eingestellt.

Das Optimum der Reaktionsgeschwindigkeit liegt im pH-Bereich zwischen 7,5 und 9.

Der Testansatz hatte in Verbindung mit der D-2-Hydroxy-4-methylpentansäure-Dehydrogenase folgende Zusammensetzung:
0,18 mM NADH
0,88 mM ACA
2,7 U D-2-Hydroxy-4-methylpentansäure-Dehydrogenase 0,1 M Phosphatpuffer bei unterschiedlichen pH-Werten und limitierende Mengen an ACA-Acylase.
Die gewählten pH-Werte lagen zwischen pH 6,2 und 8,0.

Das Optimum der Reaktionsgeschwindigkeit liegt im pH-Bereich zwischen 6,5 und 7,8.

Beispiel 5: Optimale Temperatur

Die Temperatur-Abhängigkeit der hydrolytischen Abspaltung von Essigsäure aus der Verbindung α-Acetylaminozimtsäure wurde in Gegenwart der ACA-Acylase und von D-2-Hydroxy-4-methylpentansäure-Dehydrogenase untersucht.

Der Testansatz für die Hydrogenierung des Phenylpyruvates hatte folgende Zusammensetzung:
0,23 mM NADH
5 mM ACA
0,1 M Phosphatpuffer für pH 7,5

Dieses Reagenzgemisch wurde 10 Minuten bei verschiedenen Temperaturen zwischen 25 °C und 60 °C inkubiert, jeweils mit 2,5 U D-2-Hydroxy-4-methylpentansäure-Dehydrogenase sowie limitierenden Mengen an ACA-Acylase versetzt und dann wurde die Geschwindigkeit der Reaktion bei der gwählten Inkubationstemperatur in einem Spektralphotometer gemessen. Die maximale Reaktionsgeschwindigkeit wurde bei 52 °C erreicht und ist damit um den Faktor 2,5 höher als bei der Standardtemperatur von 30 °C.

Beispiel 6: pH-Stabilität der ACA - Acylase

Die pH-Stabilität der ACA-Acylase wurde bei pH-Werten im Bereich von 5,0 bis 11,0 untersucht. Das Enzym wurde für definierte Zeiten bei verschiedenen pH-Werten gehalten und danach die ACA-Acylase-Aktivität unter Standardbedingungen bei 30 °C bestimmt. Experimentell wurde das Enzym 1:11 mit 100 mM an Puffern, die verschiedene pH-Werte hatten, verdünnt. Für diese verschiedenen pH-Bereiche wurden folgende Puffer eingesetzt:

| | |
|---|---|
| 100 mM Citronensäure/NaOH | pH 5,0; 5,5; 6,0 |
| Kaliumphosphat | pH 6,0; 6,5; 7,0; 7,5; 8,0 |
| Tris/HCl | pH 8,0; 8,5; 9,0 |
| $NH_4Cl/NH_4OH$ | pH 9,0; 9,5; |
| Glycin/NaCl/NaOH | pH 10,0; 10,5; 11,0; 11,5 |

Standardbedingungen:

0,23 mM NADH
5 mM ACA
0,1 M Phosphatpuffer für pH 7,5
2,5 U D-2-Hydroxy-4-metylpentansäure-Dehydrogenase limitierende Mengen an ACA-Acylase

Die nachfolgende Tabelle 4 zeigt die prozentuale Restaktivität der ACA-Acylase nach 1 Stunde, 1 Tag, 1 Woche bzw. 2 Wochen.

Nach 2 Wochen konnten im pH-Bereich von 6,9 bis 9,4 noch über 90 % der ursprünglichen ACA-Acylase-Aktivität nachgewiesen werden.

Tabelle 4

| pH-Stabilität der ACA-Acylase | | | | | |
|---|---|---|---|---|---|
| Puffer 0,1 M | gemessener pH-Wert | (%) Restaktivität nach | | | |
| | | 1 Stunde | 1 Tag | 1 Woche | 2 Wochen |
| Citronensäure/ NaOH | 4,95 | 26 | 2 | - | - |
| | 5,35 | 86 | 17 | 5 | - |
| | 5,85 | 99 | 69 | 20 | 11 |
| Kaliumphosphat | 5,95 | 99 | 75 | 34 | 14 |
| | 6,45 | 99 | 99 | 96 | 54 |
| | 6,9 | 99 | 99 | 99 | 93 |
| | 7,4 | 99 | 99 | 99 | 90 |
| | 7,85 | 99 | 99 | 99 | 75 |
| Tris/HCl | 7,9 | 99 | 99 | 98 | 93 |
| | 8,0 | 99 | 99 | 94 | 94 |
| | 9,12 | 99 | 99 | 86 | 90 |
| $NH_4Cl/NH_4OH$ | 8,95 | 99 | 99 | 79 | 93 |
| | 9,42 | 99 | 99 | 87 | 91 |
| Glycin/NaCl/ NaOH | 10,0 | 99 | 99 | 90 | 80 |
| | 10,3 | 99 | 99 | 90 | 5 |
| | 10,7 | 93 | 52 | 18 | - |
| | 11,1 | 44 | 5 | - | - |

Beispiel 7: Temperaturbeständigkeit der ACA-Acylase

Die Temperaturbeständigkeit der ACA-Acylase wurde in einem Temperaturbereich von 25 °C bis 60 °C untersucht. Das Enzym wurde 10, 20 und 30 Minuten bei der gewählten Temperatur in Phosphatpuffer (pH 7,5) inkubiert. Danach wurde die verbliebene ACA-Acylase-Aktivität unter Standardbedingungen bei 30 °C ermittelt.

Standardbedingungen:

0,23 mM NADH
5 mM ACA
0,1 M Phosphatpuffer für pH 7,5
2,5 U D-2-Hydroxy-4-methylpentansäure-Dehydrogenase limtierende Mengen an ACA-Acylase.

Nach 30 minütiger Inkubation bei 55 °C sind noch 80 % der Ausgangsaktivität nachzuweisen, bei weiter zunehmender Inkubationstemperatur wird die ACA-Acylase schnell denaturiert.

Beispiel 8: Einflüsse von Inhibitoren und Aktivatoren

Der Einfluß verschiedener Inhibitoren und Aktivatoren auf die Aktivität der ACA-Acylase und die daraus resultierende Reaktionsgeschwindigkeit der hydrolytischen Abspaltung von Essigsäure aus der Verbindung α-Acetylaminozimtsäure wurde unter Standardbedingungen, wie im Beispiel 7 beschrieben, gemessen. (Abweichung: Die Versuche mit 2-wertigen Metallionen wurden in 0,1 M Tris/HCl-Puffer für pH 8,0 durchgeführt).

Die Ergebnisse sind in Tabelle 5 zusammengefaßt.

Tabelle 5

| Einflüsse von Inhibitoren und Aktivatoren | | |
|---|---|---|
| Reagenz | Konzentration (mM) | Restaktivität (%) |
| ohne Zusatz | 0 | 100 |
| o-Phenanthrolin | 1 | 72 |
| | 10 | 45 |
| 2,2-Dipyridyl | 1 | 97 |
| | 10 | 69 |
| EDTA | 1 | 111 |
| | 10 | 102 |
| p-Mercuribenzoat | 1 | 41 |
| | 10 | 3 |
| Dithiothreitol | 1 | 146 |
| | 10 | 123 |
| 2-Mercaptoäthanol | 1 | 100 |
| | 10 | 100 |
| red. Glutathion | 1 | 102 |
| | 10 | 81 |
| $MgCl_2$ | 1 | 100 |
| | 10 | 100 |
| $MnCl_2$ | 1 | 63 |
| $CaCl_2$ | 1 | 99 |
| | 10 | 99 |
| $CdCl_2$ | 1 | 72 |
| | 10 | 29 |
| $HgCl_2$ | 1 | 51 |
| | 10 | 0 |
| $NiCl_2$ | 1 | 83 |
| | 10 | 53 |
| $ZnCl_2$ | 1 | 86 |
| | 10 | 34 |

Beispiel 9: Bestimmung des Molekulargewichts der ACA-Acylase und Ermittlung der Untereinheiten

Das Molekulargewicht des nativen Enzyms wurde durch Gelfiltration an Superose 12 ermittelt. Die an ein FPLC-System (Firma Pharmacia, Uppsala, Schweden) gekoppelte Säule (1,0 × 30 cm) wurde mit einer Durchflußrate von 0,4 ml/min betrieben, wobei als Probegut 75 $\mu$l des an Mono Q gereinigten Enzyms dienten.

Als Eichproteine wurden Cytochrom C, Pepsin, Ovalbumin, Rinderserumalbumin (BSA), D-2-Hydroxyisocaproat-Dehydrogenase, L-2-Hydroxyisocaproat-Dehydrogenase, Aldolase, L-Alanin-Dehydrogenase und L-Leucin-Dehydrogenase aus B. cereus und Ferritin verwendet.

Das Molekulargewicht der ACA-Acylase beträgt 50 000 ± 5000 Dalton.

Durch Gelelektorphorese in Gegenwart von Natriumdodecylsulfat (SDS) konnte die Größe und Anzahl der Untereinheiten des Enzyms bestimmt werden. Das Molekulargewicht der Untereinheiten beträgt 26 000 ± 3000 Dalton. Das bedeutet, daß die ACA-Acylase aus 2 identischen Untereinheiten besteht. Für die Eichkurve wurde Hämoglobin, $\beta$-Lactoglobulin, Chymotrypsinogen A, Pepsin, Ovalbumin und BSA verwendet.

Beispiel 10: Abhängigkeit der ACA-Acylase-Aktivität von der Substratkonzentration

a) Die Abhängigkeit der Reaktionsgeschwindigkeit der hydrolytischen Abspaltung von Essigsäure aus der Verbindung $\alpha$-Acetylaminozimtsäure in Gegenwart der ACA-Acylase und nachfolgenden reduktiven Aminierung in Gegenwart von L-Phenylalanin-Dehydrogenase zu L-Phenylalanin wurde in folgendem

Testansatz untersucht:
0,7 mM Ammoniumchlorid/Ammoniak-Puffer (pH 8,5)
0,23 mM NADH
0,5 U L-Phenylalanin-Dehydrogenase,
limitierende Mengen an ACA-Acylase.
Die α-Acetylaminozimtsäure-Konzentration wurde im Bereich von 1 - 36 mM variiert. Es zeigt sich, daß die optimale Reaktionsgeschwindigkeit bei 16 mM erreicht wird. Der $K_M$-Wert für α-Acetylaminozimtsäure beträgt 3,9 mM.
b) Die Abhängigkeit der Reaktionsgeschwindigkeit der hydrolytischen Abspaltung von Essigsäure aus der Verbindung α-Acetylaminozimtsäure in Gegenwart der ACA-Acylase und nachfolgenden Hydrogenierung des Phenylpyruvates in Gegenwart von D-2-Hydroxy-4-methylpentansäure-Dehydrogenase zu D-Phenylmilchsäure wurde in folgendem Testansatz untersucht:
0,1 M Phosphatpuffer (pH 7,5)
0,23 mM NADH
0,8 U D-2-Hydroxy-4-methylpentansäure-Dehydrogenase limitierende Mengean an ACA-Acylase.
Die α-Acetylaminozimtsäure-Konzentration wurde im Bereich von 0,1 - 18 mM variiert. Es zeigte sich, daß die optimale Reaktionsgeschwindigkeit bei 5 mM erreicht wird. Der KM-Wert für α-Acetylaminozimtsäure liegt bei 0,45 mM.

Die Messungen zeigen, daß der $K_M$-Wert vom pH-Uert abhängt: Bei pH 7,5 beträgt er 0,45 mM, bei pH 8,5 liegt er bei 3,9 mM. Zur Charakterisierung des Enzyms sollte der Wert genommen werden, der im pH-Optimum bestimmt wurde, d. h. der $K_M$-Wert für das Substrat ACA liegt bei 0,45 mM (pH 7,5).

Beispiel 11: Substratspezifität der ACA-Acylase

In einer Reihe paralleler Ansätze wurde das Substrat α-Acetylaminozimtsäure durch andere Verbindungen ersetzt und geprüft, inwieweit das Enzym andere Substrate akzeptiert.
Folgende Testansätze wurden - je nach eingesetztem Substrat - dabei verwendet:
1. Test mit α-Acetylaminozimtsäure als Substrat:
0,7 M $NH_4Cl$
0,1 mM NADH
50 mM α-Acetylaminozimtsäure
1 U/ml L-Phenylalanin-Dehydrogenase
0,1 U/ml ACA-Acylase
pH 9,2
Die Aktivität wurde durch einen photometrischen Test, wie in Beispiel 1 beschrieben, gemessen (bei 340 nm).
2. Test mit α-Acetylaminoacrylsäure:
0,7 M $NH_4Cl$
0,1 mM NADH
50 mM α-Acetylaminoacrylsäure
1 U/ml L-Alanin-Dehydrogenase
0,1 U/ml ACA-Acylase
pH 8,0
Die Aktivität wurde in analoger Weise durch einen photometrischen Test (bei 340 nm) gemessen (1 µMol NADH-Abnahme entspräche 1 µMol Alanin-Bildung).
3 Test mit Peptiden:
Als weitere Substrate wurden N-Acetyl-L-Phenylalanin, N-Methoxycarbonyl-D,L-Phenylalanin und verschiedene Peptide eingesetzt Die verwendeten Peptide sind in Tabelle 6 aufgeführt. Der Testansatz enthielt in diesen Fällen:
0,1 M Kaliumphosphat-Puffer (pH 7,5)
0,1 U/ml ACA-Acylase (17,8 U/mg)
50 mM Substrat
Die Ansätze (1ml gesamt) wurden gerührt (30 °C) und zu verschiedenen Zeiten Proben entnommen.
Die durch Acylase-Einwirkung entstehenden Aminosäuren wurden mit Hilfe eines Aminosäure-Analysators quantitativ erfaßt. Die Aktivität des Enzyms wird auch in diesem Fall in internationalen Einheiten angegeben, es entspricht eine Aktivität von 1 U der Bildung von 1 µMol Aminosäure pro 1 Minute.
Die mit den 3 Testansätzen gemessenen Aktivitäten sind in der Spalte 2 der Tabelle 6 in mU/ml aufgeführt. Für die Bestimmung der relativen Aktivität (Spalte 3 der Tabelle 6) wurde die Aktivität

gegenüber dem Substrat α-Acetylaminozimtsäure gleich 100 % gesetzt.

Tabelle 6: Substratspezifität der ACA-Acylase

| Substrat | Aktivität ($mU.ml^{-1}$) | relative Aktivität (%) |
|---|---|---|
| Aminosäure-derivate: | | |
| α-Acetylaminozimtsäure | 125 | 100 |
| α-Acetylaminoacrylsäure | 0 | 0 |
| N-Acetyl-L-phenylalanin | 0 | 0 |
| N-Methoxycarbonyl-D,L-phenylalanin | 0 | 0 |
| Peptide: | | |
| Gly-L-phe | 40 | 32 |
| Gly-D-phe | 0 | 0 |
| Gly-gly-L-phe | 0 | 0 |
| L-Ala-L-phe | 105 | 84 |
| L-Leu-L-phe | 102 | 82 |
| L-Phe-L-phe | 41 | 33 |
| L-His-L-phe | 44 | 35 |
| L-Ser-L-phe | 36 | 29 |
| L-Asp-L-phe | 38 | 30 |
| L-Leu-L-tyr | 109 | 87 |
| L-Leu-L-trp | 67 | 54 |
| L-Ala-L-trp | 66 | 53 |
| L-Ala-L-tyr | 78 | 62 |
| L-Ala-L-his | 43 | 34 |

Beispiel 12: Herstellung von L-Phenylalanin

Koppelt man die ACA-Acylase mit L-Phenylalanin-Dehydrogenase, kann das entstehende Zwischenprodukt durch reduktive Aminierung stereospezifisch zu L-Phenylalanin umgesetzt werden. Um das bei der Dehydrogenierungs-Reaktion oxidierte Coenzym zu regenerieren, werden dem Ansatz Formiat-Dehydrogenase (E.C.1.2.1.2) und Formiat zugesetzt. Als zusätzliches Produkt erhält man dann $CO_2$.

Es wurden verschiedene Ansätze mit unterschiedlichen Konzentrationen an ACA inkubiert, die Ansätze enthielten im einzelnen:

400 mM Ammoniumformiat (pH 9,2)
200 mM Tris/HCl (pH 9,2)
0,3 mM NADH
1 U/ml Formiat-Dehydrogenase (Präparat gemäß Kroner et al. (1982) J. Chem. Technol. Biotechnol. 32, 130-137)
1 U/ml L-Phenylalanin-Dehydrogenase (Präparat mit 44 U/mg)
0,5 U/ml ACA-Acylase
20-300 mM ACA

Das Gesamtvolumen betrug 1 ml, die Inkubation erfolgte unter Rühren bei 28 °C. Die Produktbildung

wurde am Aminosäure-Analysator verfolgt.

Die Tabelle 7 zeigt, daß in allen Ansätzen ein praktisch vollständiger Umsatz des Substrats zum Phenylalanin erfolgte.

Tabelle 7

| Umsetzung verschiedener Mengen von ACA zu L-Phenylalanin | | | |
|---|---|---|---|
| Ansatz | α-Acetylaminozimtsäure (mM) | Reaktionszeit (Std) | L-Phenylalanin (mM) |
| 1 | 20 | 4 | 21 |
| 2 | 50 | 6,5 | 56 |
| 3 | 100 | 6,5 | 98 |
| 4 | 200 | 30 | 204 |
| 5 | 300 | 75 | 306 |

Im Ansatz 5 mit 300 mM Substrat wurde nach 75 Stunden Reaktion die optische Reinheit des gebildeten L-Phenylalanins überprüft. In diesem Ansatz lag ein Teil des Produktes in unlöslicher Form als Kristallsuspension vor (Löslichkeit von Phe in $H_2O$ bei 25 °C = 180 mM). Um das gesamte Produkt in Lösung zu bringen, wurden dem Ansatz 3 ml $H_2O$ zugesetzt. Durch Gelfiltration an Sephadex G-25 (Pharmacia) wurden die Proteine entfernt und die Fraktion mit den niedermolekularen Substanzen wurde nach Gefriertrocknung mit 4,5 ml $H_2O$ aufgenommen.

Ein Test am Aminosäure-Analysator ergab einen L-Phenylalanin-Gehalt von 37,4 mM. Ein Test mit L-Aminosäure-Oxidase ergab 37 mM L-Phe. Ein Test mit D-Aminosäure-Oxidase war negativ, wobei die Nachweisgrenze bei 0,04 mM D-Phe liegt.

Beispiel 13: Herstellung von D-Phenylmilchsäure (kontinuierlich)

ACA kann enzymatisch zu D-Phenylmilchsäure umgesetzt werden, indem die ACA-Acylase mit einer D-2-Hydroxysäure-Dehydrogenase gekoppelt wird. Als Dehydrogenase kann eine entsprechende D-Lactat-Dehydrogenase (z.B. aus Lactobacillus confusus, s. Hummel, W. et al., Eur. J. Appl. Microbiol. Biotechnol. 18:75-85 (1983) ) oder eine D-2-Hydroxy-4-methylpentansäure -Dehydrogenase verwendet werden. Die Coenzym-Regenerierung erreicht man in diesem Beispiel in Gegenwart von Formiat mit einer Formiat-Dehydrogenase.

Der Ansatz (10 ml Gesamtvolumen) enthielt im einzelnen:

400 mM Natriumformiat (pH 7,5)
200 mM Tris/HCl (pH 7,5)
0,3 mM PEG 20 000-NADH
1 U/ml Formiat-Dehydrogenase
1 U/ml D-2-Hydroxy-4-methylpentansäure-Dehydrogenase (Präparat mit 270 U/mg)
0,5 U/ml ACA-Acylase (Präparat mit 6 U/mg)
20 mM ACA

Eine kontinuierliche Umsetzung erreicht man in einem Enzym-Membran-Reaktor. Dazu wird der Reaktionsansatz über eine Ultrafiltrations-Membran gepumpt (Typ YM5, Firma Amicon, Ausschlußgrenze 5 000 Dalton). Niedermolekulare Bestandteile können dabei kontinuierlich aus der Reaktionsmischung entfernt werden (Verweilzeit 3 Stunden). Das Volumen der ultrafiltrierten Produktlösung wurde in dieser Versuchsanordnung kontinuierlich durch Substratlösung ersetzt. Die Substratlösung enthielt:

400 mM Natriumformiat (pH 7,5)
200 mM Tris/HCl (pH 7,5)
20 mM ACA

Der Umsetzungsgrad wurde bestimmt, indem der Drehwert α der Produktlösung polarimetrisch gemessen wurde. Die Produktkonzentration kann dann aus einer Eichkurve, die mit käuflicher D-Phenylmilchsäure (Sigma) erstellt wurde, ermittelt werden.

Tabelle 8 zeigt, daß man ACA praktisch vollständig zu D-Phenylmilchsäure umsetzen kann.

Tabelle 8

| Umsetzung von ACA (20 mM) zu D-Phenylmilchsäure | | | |
|---|---|---|---|
| Zeit (Stunden) | Drehwert (Grad) | Produktkonzentration mM | Umsatz % |
| 5 | 202 | 12,1 | 61 |
| 10 | 305 | 18,1 | 91 |
| 15 | 330 | 20 | 100 |
| 20 | 330 | 20 | 100 |
| 25 | 330 | 20 | 100 |

## Patentansprüche

1. Mikrobiologisch hergestellte α-Acetylaminozimtsäure-Acylase, gekennzeichnet durch die folgenden Eigenschaften:

   1) Reaktivität:
   sie reagiert mit α-Acetylaminozimtsäure unter Bildung von α-Imino-β-phenyl-propionsäure bzw. Phenylbrenztraubensäure;
   2) Subtratspezifität:
   sie hydrolysiert α-Acetylaminozimtsäure, nicht aber andere α-Acetylaminocarbonsäuren, wie α-Acetylaminoacrylsäure oder N-Acetylphenylalanin;
   3) Optimaler pH-Wert:
   der optimale pH-Wert ist 7,5 ± 1;
   4) pH-Stabilität:
   sie zeigt eine gute Stabilität im pH-Bereich zwischen 6,9 und 9,4 (mehr als 90 % Restaktivität nach 2 Wochen bei 4 °C);
   5) Optimale Temperatur:
   die optimale Temperatur beträgt 52 °C bei einem pH-Wert on 7,5;
   6) Temperaturbeständigkeit:
   bei 55 °C sind nach 30-minütiger Inkubation noch 80 % Restaktivität nachweisbar;
   7) Einflüsse von Inhibitoren und Aktivatoren:
   inhibierend wirken vor allem Quecksilber-Verbindungen (p-Mercuribenzoat und $HgCl_2$),$CdCl_2$ und o-Phenanthrolin, Zusatz von Dithiothreitol (1 mMol) aktiviert das Enzym;
   8) Molekulargewicht:
   das Molekulargewicht beträgt 50 000 ± 5000 Dalton;
   9) Untereinheiten:
   das Molekül besteht aus 2 gleichgroßen Untereinheiten mit je 26 000 ± 3000 Dalton;
   10) der $K_M$-Wert für das Substrat α-Acetylaminozimtsäure bei pH 7,5 ist 0,45 mMol.

2. Verfahren zur Gewinnung der α-Acetylaminozimtsäure-Acylase gemäß Anspruch 1, dadurch gekennzeichnet, daß man Brevibakterium spec. NCIB 12246 oder NCIB 12247 in einem wäßrigen Nährmedium, das eine Quelle für Kohlenstoff und Stickstoff, Thiamin, Mineralsalze und α-Acetylaminozimtsäure als Induktor enthält, bei einem Ausgangs-pH-Wert zwischen 6,0 und 8,0 und einer Temperatur zwischen 25 °C und 35 °C aerob kultiviert, die Zellmasse abtrennt und das Enzym aus den Zellen isoliert.

3. Verwendung der α-Acetylaminozimtsäure-Acylase gemäß Anspruch 1 als Bestandteil eines gekoppelten Enzymsystems für enzymatische Umsetzungen, die über die Zwischenstufe α-Imino-β-phenyl-propionsäure bzw. Phenylbrenztraubensäure verlaufen.

## Claims

1. Microbiologically prepared α-acetylaminocinnamic acid acylase, characterized by the following properties:
   1) reactivity:

it reacts with α-acetylaminocinnamic acid to form α-imino-β-phenylpropionic acid or phenylpyruvic acid;
2) substrate specificity:
it hydrolyzes α-acetylaminocinnamic acid, but not other α-acetylaminocarboxylic acids, such as α-acetylaminoacrylic acid or N-acetyl phenylalanine;
3) optimal pH value:
the optimal pH value is 7.5 ± 1;
4) pH stability:
it shows high stability in the pH range from 6.9 to 9.4 (more than 90% residual activity after 2 weeks at 4 °C);
5) optimal temperature:
the optimal temperature is 52 °C for a pH of 7.5;
6) heat stability:
at 55 °C, 80% residual activity can still be detected after incubation for 30 minutes;
7) influences of inhibitors and activators:
the enzyme is inhibited above all by mercury compounds (p-mercury benzoate and $HgCl_2$), $CdCl_2$ and o-phenanthroline, addition of dithiothreitol (1 mmol) activates the enzyme;
8) molecular weight:
the molecular weight is 50,000 ± 500 dalton;
9) sub-units:
the molecule consists of 2 equally large sub-units each having a molecular weight of 26,000 ± 3,000 dalton;
10) the $K_M$ value for the substrate α-acetylaminocinnamic acid at pH 7.5 is 0.45 mmol.

**2.** A process for recovery of the α-acetylaminocinnamic acid acylase claimed in claim 1, characterized in that Brevibacterium spec. NCIB 12246 or NCIB 12247 is aerobically cultivated in an aqueous nutrient medium containing a source of carbon and nitrogen, thiamine, mineral salts and α-acetylaminocinnamic acid as inductor at a starting pH value of 6.0 to 8.0 and at a temperature of 25 °C to 35 °C, the cell mass is removed and the enzyme is isolated from the cells.

**3.** The use of the α-acetylaminocinnamic acid acylase claimed in claim 1 as part of a coupled enzyme system for enzymatic reactions which proceed via the intermediate stage of α-imino-β-phenylpropionic acid or phenyl pyruvic acid.

## Revendications

**1.** Acide α-acétylaminocinnamique-acylase obtenue par voie microbiologique, caractérisée par les propriétés suivantes :
1 - Réactivité :
Elle réagit avec l'acide α-acétylaminocinnamique avec formation d'acide α-imino-β-phénylpropionique ou d'acide phénylpyruvique,
2 - Spécificité du substrat :
Elle hydrolyse l'acide α-acétylaminocinnamique, mais pas d'autres acides α-acétylamino carboxyliques, comme l'acide α-acétylaminoacrylique ou la N-acétylphénylalanine,
3 - Valeur du pH optimale :
la valeur optimale du pH est 7,5 ± 1,
4 - Stabilité au pH :
Elle montre une bonne stabilité dans la zone de pH comprise entre 6,9 et 9,4 (plus de 90 % d'activité résiduelle après 2 semaines à 4 °C),
5 - Température optimale :
la température optimale s'élève à 52 °C pour une valeur de pH de 7,5,
6 - Stabilité à la température :
à 55 °C après 30 mn d'incubation, on peut encore mettre en évidence 80 % d'activité résiduelle,
7 - Influences des inhibiteurs et des activateurs :
avant tout ce sont les composés du mercure (p-mercuribenzoate et $Cl_2Hg$), $Cl_2Cd$ et l'o-phénanthroline qui agissent comme inhibiteur, l'addition de dithiothreitol (1 mmol) active l'enzyme,
8 - Poids moléculaire :
le poids moléculaire s'élève à 50.000 ± 5000 daltons,

9 - Sous unités :
la molécule se compose de 2 sous-unités de même grandeur ayant chacune 26.000 ± 3000 daltons,
10 - La valeur du Km pour le substrat acide α-acétylaminocinnamique à pH 7,5 est 0,45 mmol.

**2.** Procédé pour la production de l'acide α-acétylaminocinnamique-acylase selon la revendication 1, caractérisé en ce que l'on met en culture en érobie, Brevibacterium spec. NCIB 12246 ou NCIB 12247 dans un milieu nutritif aqueux qui renferme une source pour le Carbone et l'Azote, de la thiamine, des sels minéraux et de l'acide α-acétylaminocinnamique comme inducteur, à une valeur initiale de pH comprise entre 6,0 et 8,0 et à une température comprise entre 25 °C et 35 °C, que l'on sépare la masse cellulaire et que l'on isole l'enzyme des cellules.

**3.** Utilisation de l'acide α-acétylaminocinnamique-acylase, selon la revendication 1 comme constituant d'un système d'enzymes couplé, pour des conversions enzymatiques qui se déroulent par l'étape intermédiaire de l'acide α-imino-β-phénylpropionique ou de l'acide phénylpyruvique.